# EUROPÄISCHE PATENTSCHRIFT

(11) **EP 3 378 457 B1**
(45) Veröffentlichungstag und Bekanntmachung des Hinweises auf die Patenterteilung: **27.01.2021**
(21) Anmeldenummer: 18162519.5
(22) Anmeldetag: 19.03.2018
(51) Int. Cl.: A61G 9/00, A61F 13/42

(54) **SENSORTEXTIL**
SENSOR TEXTILE
TEXTILE CAPTEUR

(30) Priorität: 21.03.2017 AT 502282017
(43) Veröffentlichungstag der Anmeldung: 26.09.2018
(73) Patentinhaber: Texible GmbH, 6850 Dornbirn (AT)
(72) Erfinder: Bechtold, Thomas, 6850 Dornbirn (AT); Bösch, Guntram, 6890 Lustenau (AT); Fröis, Josef, 6850 Dornbirn (AT); Fröis, Thomas, 6833 Weiler (AT); Grabher, Günter, 6890 Hohenems (AT); Scheiderbauer, Manuel, 6850 Dornbirn (AT)
(74) Vertreter: Schwarz & Partner Patentanwälte OG

(56) Entgegenhaltungen:
- EP-A1- 3 315 955
- US-A1- 2013 167 296
- US-A1- 2015 045 630
- US-B1- 9 506 886

## Beschreibung

Die Erfindung betrifft ein Sensortextil, umfassend textile Lagen. Weiters betrifft die Erfindung eine Vorrichtung zur Nässedetektion in textilen Strukturen, Betten oder Windeln. Außerdem betrifft die Erfindung eine Vorrichtung zur Ermittlung der Lageposition eines Subjekts in einem Bett. Schließlich betrifft die Erfindung eine Anordnung aus Vorrichtung zur Nässedetektion und Vorrichtung zur Ermittlung der Lageposition eines Subjekts in einem Bett.

### Gebiet der Erfindung

Bei der Betreuung inkontinenter Personen kommt der Überwachung des Nässezustands im Bettzeug große Bedeutung zu. Zur Verhinderung eines regelmäßigen Einnässens werden bei pflegebedürftigen Personen Einlagen verwendet, welche in der Lage sind, auch große Mengen an Harn aufzunehmen. Tritt jedoch durch Verrutschen oder Hantieren der Betreuten ein Verschieben der Einlage auf, kommt es zu einem Nässeaustritt und die betreute Person liegt für einen längeren Zeitraum nass.

Gerade bei älteren Personen ist längeres Liegen in nasser Umgebung für die bereits geschwächte Haut sehr ungünstig, da es in weiterer Folge zu Infektionen und schlecht heilende Entzündungen kommen kann. Um diesem Umstand zu begegnen, werden in Pflegeheimen regelmäßige Kontrollen angeordnet, damit längeres Liegen in nassen Betttextilien vermieden wird. Diese Kontrollen finden insbesondere in der Nacht statt, wodurch ein hoher Pflegeaufwand verursacht und die Schlafqualität der Betreuten beeinträchtigt wird. Da solche Nässekontrollen oft manuell durch Eingriff in die Betthöhle durchgeführt werden, ergibt sich für das Pflegepersonal und die Betreuten eine zusätzliche Belastung.

Um für den Fall eines Einnässens die Hygiene der Matratze zu gewährleisten, wird auf die Matratze ein sogenannter Hygieneschutz aufgebracht. Dieser ist in den meisten Fällen eine waschbare, dicke und flüssigkeitsundurchlässige Einlage, welche in der Lage ist, auch eine gewisse Menge an Nässe zu speichern. Die dafür erforderliche Dicke der Einlage vermindert die Wirkung einer darunter befindlichen Anti-Dekubitus-Matratze, sie ist jedoch erforderlich, um eine zumindest begrenzte Nässespeicherung zu gewährleisten. Zusätzlich sind diese relativ dicken Einlagen mit hohem Trocknungsaufwand bei der Wäsche verbunden, da sie eine hohe Wasseraufnahmekapazität aufweisen und beim Reinigen mit viel Wasser beladen werden.

Ein anderes Problem in der Pflege - insbesondere von mental eingeschränkten Personen - ergibt sich aus der Positionsüberwachung zur Vorbeugung von Stürzen, der sogenannten Sturzprophylaxe. Um Stürze beim unkontrollierten Aufstehen bzw. Verlassen des Pflegebettes zu vermeiden, wäre eine frühe Erkennung einer Positionsveränderung der Person - idealerweise noch während der Aufstehphase - wünschenswert.

### Stand der Technik

Nach DE 600 22 515 wird zur Inkontinenzdetektion ein Sensorelement vorgeschlagen, welches in einer Sandwichstruktur aus leitenden und nichtleitenden Strukturen einen Schalter bildet, welcher bei Vorhandensein von Nässe elektrisch leitend wird und damit als Auslöser für eine Signalanordnung dienen kann. Aufgrund der Konstruktion als sandwichförmiger Schalter werden dabei nennenswerte Flüssigkeitsmengen zwischen den Schalterplatten aufgenommen was eine Hygenisierung und Wäsche nachteilig erschwert. Ebenso ist die bevorzugte Anordnung (Seite 3) durch Verwendung von Aluminium als leitendes Metall hergestellt, was eine Wäsche unter üblichen Bedingungen einer Hygienewäsche (mind. 60°C, Vollwaschmittel) aus Korrosionsgründen ausschließt. US 2013/0167296 A1 offenbart einen Bettüberzug zur Inkontinez-Überwachung. US 2015/0045630 A1 bezieht sich auf eine Einrichtung zur Druckmessung mithilfe einer Vielzahl von kapazitiven Elementen. US 9 506 886 B1 offenbart ein System zur Detektion von Flüssigkeiten.

### Kurzbeschreibung der Erfindung

Im Stand der Technik werden verschiedene Systeme beschrieben, bei denen Inkontinenz durch Leitwertmessung ermittelt wird. Mehrheitlich sind die Detektionseinheiten nicht waschbar, da die Aufbauten der mechanischen und chemischen Belastung einer Hygienewäsche nicht standhalten. Die wenigen bekannten waschbaren Inkontinenzdetektionssysteme für Pflegebetten haben mehrere Nachteile. Insbesondere weisen diese eine Vielzahl an Schichten auf, sodass sie aufwändig in der Herstellung und im Bett aufgrund der Dicke störend sind. Aufgabe der vorliegenden Erfindung ist daher die Bereitstellung einer dünnen Einheit zur Nässedetektion für Pflegebetten, welche einer hohen Temperatur beim Reinigen (Kochwäsche) standhält und damit für Pflegeheime einsetzbar ist.

Gelöst wird diese Aufgabe durch eine Vorrichtung mit den Merkmalen des Anspruchs 1. Die Vorrichtung umfasst ein Sensortextil, wobei das Sensortextil ein Laminat, mit
i) einer oberen Lage aus einem textilen Oberstoff,
ii) einer unter der oberen Lage angeordneten textilen Zwischenlage welche an der dem Oberstoff zugewandten Seite leitfähige Sensorfäden, vorzugsweise als Stickerei, aufweist,
iii) einer unter der Zwischenlage angeordneten Barrierelage,
iv) einer darunter angeordneten unteren Lage aus einem textilen Unterstoff umfasst.

Die Oberseite des Sensortextils umfasst eine Zwischenlage, die mit leitfähigen Sensorfäden versehen ist. Mit der Stickmaschine wird zuerst ein Stoff mit dem leitfähigen Faden auf der Vorderseite der Zwischenlage bestickt.

Darauf ist der Oberstoff appliziert und mit einem textilen Zwirn mit dem Sensorstoff durch ein Stickmuster verbunden. Der Oberstoff kann z.B. ein weicher, hautfreundlicher Flanell sein. Zwischen der Zwischenlage und dem Oberstoff kann noch eine weitere Zwischenlage angeordnet sein.

Die Zwischenlage hat an der der Barrierelage abgewandten Seite bzw. an der dem Oberstoff zugewandten Seite leitfähige Sensorfäden mit einer elektrisch leitenden Messstruktur.

Die Sensorfäden sind vorzugsweise als Stickerei ausgebildet. Bevorzugt besteht der Gegenfaden für die Stickerei an der der Barrierelage zugewandten Seite aus einem textilen Garn, Faden oder Zwirn.

Die Einheit zur Nässedetektion ist dabei ein Sensortextil, also ein Textil, welches durch Aufbringen von leitfähigen Sensorfäden als Sensor einsetzbar ist. Das Sensortextil ist ein mehrlagiges Laminat, welches so gestaltet ist, dass durch eine flüssigkeitsdichte Barrierelage im Inneren des Textils ein Hygieneschutz für die Matratze gegeben ist. Die Gesamtdicke dieses als Einlage konzipierten Sensortextils kann eine Gesamtdicke von nicht mehr als 3 mm aufweisen. Dadurch wird die Funktion der Anti-Dekubitus-Matratze nicht nachteilig beeinträchtigt.

Die Dicke kann dabei so gewählt werden, dass eine leichte Entwässerung bei der Wäsche und eine Hygienisierung möglich ist. Daher ist es vorteilhaft die Dicke der textilen Stofflagen so zu wählen, dass bei der Wäsche die Massen nicht so weit ansteigt, dass eine hohe mechanische Beanspruchung durch die Waschbewegung eintritt.

Die Barrierelage ist im Wesentlichen undurchlässig für flüssiges Wasser, d.h. beim Einnässen keine Flüssigkeit durch die Barrierelage treten kann. Die Barrierelage kann aber durchlässig für Wasserdampf sein, damit eine gewisse Atmungsaktivität gewährleistet ist.

Es hat sich messtechnisch als vorteilhaft erwiesen, wenn die Stickerei auf der dem Oberstoff zugewandten Seite im Wesentlichen kammförmig ausgebildet ist, da so die Messgenauigkeit hoch ist.

Bevorzugt ist dabei vorgesehen, dass die die Stickerei auf der dem Oberstoff zugewandten Seite aus einem leitfähigen Garn besteht. Als leitfähiges Garn oder Faden können beliebige Materialien aus der Gruppe der leitenden Stoffe ausgewählt werden, z.B. leitfähige Metalldrähte, Metallfasergarne, leitfähige Kunststoffe, Carbon-Materialien oder Kombinationen oder Mischungen daraus. Die Korrosionsbeständigkeit und mechanische Beständigkeit wird dabei bevorzugt so gewählt, dass eine Wäsche und Wiederverwendung des Sensortextils möglich ist. Die elektrischen Sensorfäden sind waschbar.

In einer bevorzugten Ausführungsform werden Edelstahlstapelfasergarne zum Sticken der Kammstruktur verwendet. In einer besonders bevorzugten Ausführungsform beträgt die Größe des Felds mit einer Stickerei ca. 640 x 1000 mm und die Abstände zwischen den benachbarten Kammzinken der Kammstruktur ca. 75 mm.

Bevorzugt ist vorgesehen, dass der textile Oberstoff hydrophil ausgebildet ist. Dieser kann aus den üblichen textilen Fasern z.B. Baumwolle, Polyester oder Mischungen dieser bestehen. Als Flächenmaterial kann der Oberstoff aus Maschenware oder Gewebe hergestellt werden, wobei auch Nonwovens verwendet werden können. In einer bevorzugten Ausführungsform besteht der Oberstoff aus einer intimen Mischung 50% Baumwolle / 50 % Polyester als Garn, welche durch Weben verarbeitet wird. Auf diesen Oberstoff wird die Stickerei - vorzugsweise in Kammstruktur - aufgebracht. Über die Sensorstickerei wird bevorzugt ein 100% Baumwollgewebe mit aufgerauter Oberfläche aufgebracht und mit einem Polyestergarn durch Stickerei befestigt.

Um Widerstandsänderungen durch Waschprozesse verfolgen und kompensieren zu können, kann zusätzlich eine Messschleife als Referenzstrecke auf den textilen Oberstoff aufgebracht werden, welche eine rechnerische Kompensation von Änderungen an der leitfähigen Stickerei erlaubt.

Die Leiterbahnen werden bevorzugt so an die Seite des Sensortextils geführt, dass durch die Stickerei entsprechende Kontaktpunkte ausgeführt werden können, an die die Auswerteelektronik durch bekannte Verfahren der Kontaktierung angeschlossen werden kann. Der Kontaktpunkt kann z.B. eine Steckverbindung, Crimpverbindung, Metalldruckknöpfe, Magnetkontakte oder dgl. sein. Die bevorzugte Ausführung sind Edelstahldruckknöpfe.

Weiters kann vorgesehen sein, dass der Unterstoff ein Gewebe, eine Maschenware oder ein Nonwoven ist. Ebenso können Fasermaterialien entsprechend den geforderten Eigenschaften der Anwendung aus den üblichen Materialien ausgewählt werden.

In eine bevorzugen Ausführungsform besteht der Unterstoff aus 100 % Polyestermaschenware.

Die Zwischenlage mit der Sensorstickerei und der Unterstoff werden durch übliche textile Fertigungsverfahren miteinander verbunden. Hierfür können Schmelzkleber oder Thermofasern eingesetzt werden. In einer Ausführungsvariante werden der Unterstoff und die Barrierelage laminiert und darauf der Oberstoff und die Zwischenlage, die durch Nähen der Sticken vorverbunden sind, auflaminiert.

Die Barrierelage kann z.B. selbst auch die Funktion einer Kleberschicht haben. Beispielsweise kann diese PUR (Polyurethan) sein.

Als Barrierelage kommt eine übliche flüssigkeitsdichte Membrane z.B. aus Polyurethan, PVC oder PTFE zum Einsatz. In einer bevorzugten Form wird eine Polyurethanmembran verwendet. In einer weiteren Ausführungsform kann statt einer Membran eine wasser- und luftdichte Folie aus obigen Materialien verwendet werden.

Weiters betrifft die Erfindung eine Vorrichtung zur Nässedetektion in textilen Strukturen, Betten oder Windeln, umfassend ein Sensortextil der vorgenannten Art, eine Messeinrichtung, zur Ermittlung des elektrischen Widerstands im Sensortextil und eine Ausgabeeinheit, welche das Vorhandensein von Nässe ausgibt.

Durch Messung der Impedanz der Sensoranordnung kann zusätzlich eine Information über die Position bzw. das Aufrichten und Aufstehen der betreuten Person gewonnen werden. Daher betrifft die Erfindung außerdem eine Vorrichtung zur Ermittlung der Position eines Subjekts in einem Bett, umfassend ein Sensortextil der vorgenannten Art, eine Messeinrichtung, zur Ermittlung der Impedanz im Sensortextil und eine Ausgabeeinheit, welche die Änderung der Lageposition des Subjekts ausgibt.

Schließlich betrifft die Erfindung eine Anordnung, umfassend eine Vorrichtung zur Nässedetektion und eine Vorrichtung zur Ermittlung der Lageposition eines Subjets in einem Bett.

Die jeweilige Vorrichtung kann über eine Ausgabeeinheit mit dem Sensortextil in Funkverbindung stehen. Beispielsweise könnte die Ausgabeeinheit ein Mobiltelefon sein.

Unerwarteterweise zeigte sich bei Testversuchen, dass das dünne Sensortextil neben der Nässedetektion durch Leitwertänderung in trockenem Zustand auch auf Körperdruck durch Änderung der Kapazität der Anordnung reagiert. Daher kann das trockene Sensortextil auch als Sensor zur Frühdetektion von unerwünschtem nächtlichem Aufstehen in Pflegeheimen und damit zur Sturzprophylaxe eingesetzt werden.

### Detaillierte Beschreibung der Erfindung

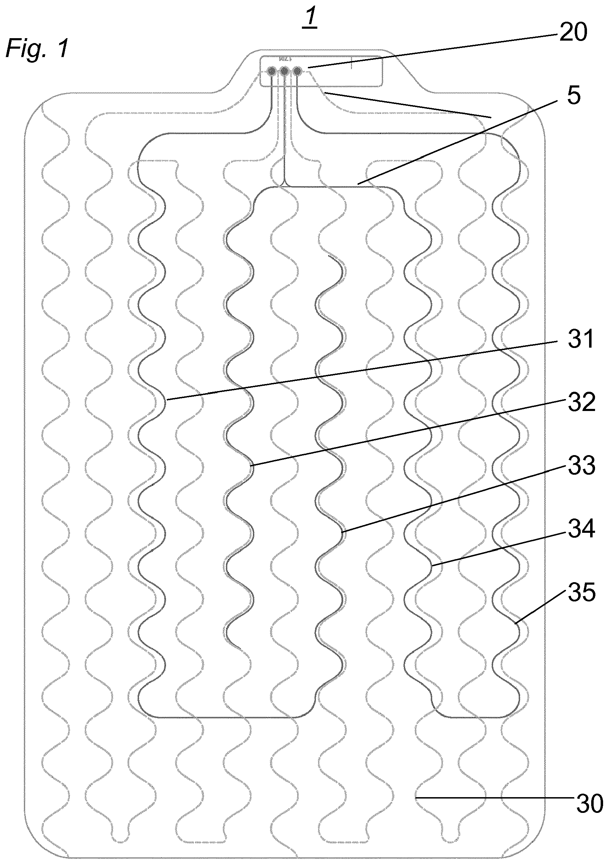

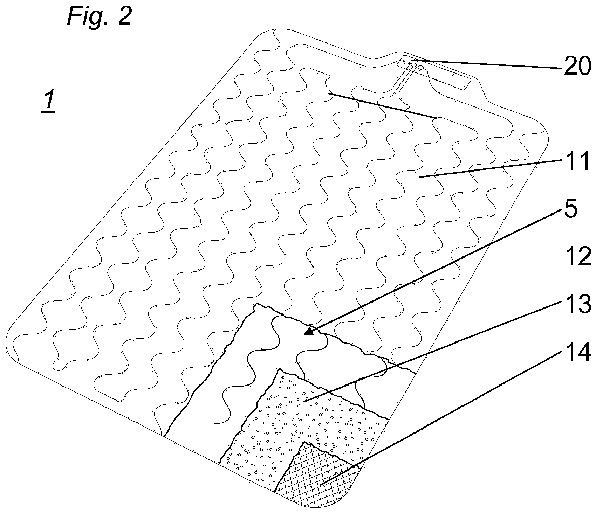

Fig. 1 und 2 zeigen ein Sensortextil in Draufsicht und in teilweise wegebrochener Ansicht.

Fig. 1 und 2 zeigen ein Sensortextil 1, umfassend ein Laminat. Dieses Laminat weist eine obere Lage aus einem textilen Oberstoff 11 und eine unter der oberen Lage angeordnete textile Zwischenlage 12 auf, welche an der dem Oberstoff 11 zugewandten Seite leitfähige Sensorfäden 5, vorzugsweise als Stickerei, aufweist. Unter der Zwischenlage 12 ist eine Barrierelage 13 angeordnet. Unter dieser Barrierelage ist eine untere Lage aus einem textilen Unterstoff 14 angeordnet. Weiters sind elektrische Kontaktpunkte 20 für die elektrisch leitfähige Stickerei vorgesehen.

An der Oberseite des Sensortextils sind wellenförmige Steppnähte 30 (strichliert) erkennbar und zur Verdeutlichung sind auch die Sensorfäden 5 durch den Oberstoff 11 sichtbar (durchgezogene Linien) dargestellt. Auch die Sensorfäden 5 sind wellenförmig ausgebildet. Dies ist vorteilhaft, wenn der Stoff verstreckt wird, da dann die Sensorfäden 5 nicht beschädigt werden. Außerdem wird die Fläche der Sensorfäden vergrößert, was zu einer besseren Messung führt, da die Belegung größer wird. Die Kammstruktur wird durch die fünf Zinken 31, 32, 33, 34, 35 gebildet. Die Zinken 34 und 35 bilden außerdem gemeinsam die Referenzstrecke.

Weiters wäre es denkbar, zwischen Zwischenlage und Barrierelage eine weitere textile Lage oder eine Vlieslage einzubringen.

Die elektrischen Kontaktpunkte 20 können z.B. als Druckknöpfe ausgebildet sein, was einen einfachen Anschluss ermöglicht. Dazu werden die Sensorfäden im Bereicht der Kontaktpunkte z.B. kreisförmig an der Zwischenlage verlegt und in der Mitte ausgespart. Dort wird anschließend ein Loch gebohrt und der Druckknopf eingesetzt.

Wird die elektronische Einheit zur Widerstandsmessung zusätzlich als Impedanzmessung ausgeführt, so kann aus dem Realteil der Messung der Ohmsche Widerstand ermittelt werden und dieser zur Auswertung als Nässesensor dienen. Aus der Erfassung des imaginären Teils der Impedanzmessung kann eine Kapazitätsveränderung beim trockenen Textil als Folge von Bewegung und Aufrichten der betreuten Person im Bett erhalten werden. Verändert sich beispielsweise die Kapazität innerhalb von weniger als 60 s um 40 % des ursprünglichen Wertes, kann eine solche Änderung als Alarm für kritische Situationen definiert werden.

Bei einem Einnässen der Stickerei aus leitfähigen Sensorfäden mit einem Volumen von 1 - 5 ml kommt es zu einer Verminderung des Ohmschen Widerstands zwischen nicht leitend verbundenen Elektroden von ursprünglich 2 - 20 giga-Ohm auf Werte im Bereich von 120 Ohm bis 1300 Ohm. Die Höhe des Ohmschen Widerstands ist dabei durch die gewählte Geometrie definiert, insgesamt ergibt sich aber eine Verminderung des Ohmschen Widerstands um den Faktor 10³ bis 10⁶, was messtechnisch gut automatisiert erfasst werden kann.

Gleichzeitig verändert sich auch die Kapazität der Anordnung. Diese ist jedoch zusätzlich auch von der Art des zwischen den Kammstrukturen befindlichen Dielektrikums abhängig. Während Luft und Betttextilien geringe Beiträge liefern, bewirkt die flächige Auflage von Personen durch die hohen Wassergehalte des Körpers bedingt eine deutliche Veränderung der Kapazität.

Durch eine automatisierte Impedanzmessung lassen sich daher Flüssigkeitsaustritt und Körperposition in vorteilhafter Weise messtechnisch erkennen und erfassen.

In einer besonders bevorzugten Ausführungsform kann daher die Kombination aus erfindungsgemäßem Sensortextil mit der am Pflegebett befestigten Auswerteelektronik
- die Alterung des Edelstahlgarns durch die Referenzschleife im Sensorgarn kompensieren
- die Nässe durch Widerstandsmessung detektieren, zwischen Schweiß und Urin unterscheiden
- und zusätzlich aus der Auswertung des Imaginärteils der am Sensortextil als Bewegungs- und Positionssensor und Warnsystem dienen.

Durch eine automatische Meldung an die Pflegestation bzw. bei Hauskrankenpflege an die Betreuungsperson kann eine rasche Einleitung von Maßnahmen gewährleistet werden.

Bei einer beispielhaften Ausführungsform wird ein Raster entsprechend Fig. 1 durch leitendes Edelstahlgarn in der Feinheit 110 tex als Kammstruktur auf eine Baumwoll/Polyester Mischgewebe mit einem Flächengewicht von 122 g/m² aufgestickt. Als Gegenfaden wird ein Polyesterzwirn (110/2 dtex) verwendet. Die Kammstruktur wird an einer Seite zu Kontaktflächen an die Seite geführt, welch anschließend zur Herstellung der Kontaktierung durch Druckknöpfe aus Edelstahl verwendet werden. Auf diese Sensorschicht wird ein Baumwollgewebe mit einem Flächengewicht von 170 g/m² appliziert und mittels Steppstichen mit der unteren Lage verbunden.

Das Einlagetextil wird durch Laminieren mit einer Membran aus Polyurethan (50 µm Dicke) und einem Unterstoff aus Baumwoll/Polyester Mischgewebe (122 g/m²) durch kaschieren verbunden.

Die Sensorfäden werden dabei auf die Innenseite der Oberen Sensorfläche gelegt. Ein Schnitt durch das Textil ist in Fig. 2 dargestellt. Über die metallischen Druckknöpfe wird eine elektrische Verbindung zur Auswerteelektronik hergestellt, welche in der Lage ist, durch Impedanzmessung den Realteil und den Imaginärteil des Widerstands des Sensortextils zu ermitteln.

## Patentansprüche

1. Vorrichtung umfassend ein Sensortextil (1), wobei das Sensortextil (1) ein Laminat, mit
i) einer oberen Lage aus einem textilen Oberstoff (11),
ii) einer unter der oberen Lage angeordneten textilen Zwischenlage (12), welche an der dem Oberstoff (11) zugewandten Seite leitfähige Sensorfäden (5), vorzugsweise als Stickerei, aufweist,
iii) einer unter der Zwischenlage (12) angeordneten Barrierelage (13), und
iv) einer darunter angeordneten unteren Lage aus einem textilen Unterstoff (14) umfasst, und eine Auswerteelektronik, **gekennzeichnet durch**
eine Messeinrichtung, zur Ermittlung des elektrischen Widerstands im Sensortextil (1) sowie zur Ermittlung der Impedanz im Sensortextil (1), wobei mit der Auswertelektronik durch Impedanzmessung der Real- und Imaginärteil des Widerstands des Sensortextils (1) ermittelbar ist.

2. Vorrichtung nach Anspruch 1, **dadurch gekennzeichnet, dass** der Gegenfaden an der der Barrierelage (13) zugewandten Seite aus einem textilen Garn besteht.

3. Vorrichtung nach Anspruch 1 oder Anspruch 2, **dadurch gekennzeichnet, dass** die Barrierelage (13) durchlässig für Wasserdampf und im Wesentlichen undurchlässig für flüssiges Wasser ist.

4. Vorrichtung nach einem der Ansprüche 1 bis 3, **dadurch gekennzeichnet, dass** die Stickerei auf der dem Oberstoff (11) zugewandten Seite im Wesentlichen kammförmig ausgebildet ist.

5. Vorrichtung nach einem der Ansprüche 1 bis 4, **dadurch gekennzeichnet, dass** die Stickerei auf der dem Oberstoff (11) zugewandten Seite aus einem leitfähigen Garn besteht.

6. Vorrichtung nach Anspruch 5, **dadurch gekennzeichnet, dass** das leitfähige Garn leitfähige Metalldrähte, leitfähige Metallfasergarne, leitfähige Kunststoffe, Carbon-Materialien oder Kombinationen bzw. Mischungen daraus umfasst.

7. Vorrichtung nach einem der Ansprüche 1 bis 6, **dadurch gekennzeichnet, dass** der textile Oberstoff (11) hydrophil ausgebildet ist, vorzugsweise aus Baumwolle, Polyester oder Mischungen daraus besteht.

8. Vorrichtung nach einem der Ansprüche 1 bis 7, **dadurch gekennzeichnet, dass** der textile Oberstoff (11) eine Maschenware, ein Gewebe oder ein Nonwoven ist.

9. Vorrichtung nach einem der Ansprüche 1 bis 8, **gekennzeichnet durch** eine zusätzliche Messschleife, vorzugsweise ist die Messschleife als Referenzstrecke aufgebracht.

10. Vorrichtung nach einem der Ansprüche 1 bis 9, **gekennzeichnet durch** elektrische Kontaktpunkte (20) für die elektrisch leitfähige Stickerei.

11. Vorrichtung nach einem der Ansprüche 1 bis 10, **dadurch gekennzeichnet, dass** der Unterstoff (14) ein Gewebe, eine Maschenware oder ein Nonwoven ist, vorzugsweise ist der Unterstoff (14) Polyestermaschenware.

12. Vorrichtung nach einem der Ansprüche 1 bis 11 zur Nässedetektion in textilen Strukturen, Betten oder Windeln, **gekennzeichnet durch** eine Ausgabeeinheit für das Vorhandensein von Nässe.

13. Vorrichtung nach einem der Ansprüche 1 bis 11 zur Ermittlung der Position eines Subjekts in einem Bett, **gekennzeichnet durch** eine Ausgabeeinheit für die Änderung der Lageposition des Subjekts.

14. Vorrichtung nach Anspruch 12 oder Anspruch 13, **dadurch gekennzeichnet, dass** die Ausgabeeinheit mit dem Sensortextil (1) in Funkverbindung steht.

15. Anordnung, umfassend eine Vorrichtung nach Anspruch 12 oder Anspruch 14 zur Nässedetektion und eine Vorrichtung ; nach Anspruch 13 oder Anspruch 14 zur Ermittlung der Position eines Subjekts in einem Bett.

## Claims

1. A device comprising a sensor textile (1), the sensor textile (1) being a laminate, comprising
i) an upper layer made of a textile top fabric (11)
ii) a textile intermediate layer (12) arranged underneath the upper layer and having conductive sensor threads (5), preferably as an embroidery, on the side facing the top fabric (11),
iii) a barrier layer (13) arranged underneath the intermediate layer (12), and
iv) a bottom layer arranged underneath and made of a textile bottom fabric (14), and an electronic evaluation unit, **characterized by**
a measuring device for determining the electrical resistance in the sensor textile (1) as well as for determining the impedance in the sensor textile (1), wherein the real and the imaginary parts of the resistance of the sensor textile (1) are determinable via an impedance measurement by means of the electronic evaluation unit.

2. A device according to claim 1, **characterized in that** the counter thread on the side facing the barrier layer (13) is made of a textile yarn.

3. A device according to claim 1 or claim 2, **characterized in that** the barrier layer (13) is permeable to water vapour and essentially impermeable to liquid water.

4. A device according to any of claims 1 to 3, **characterized in that** the embroidery on the side facing the top fabric (11) has an essentially comb-shaped design.

5. A device according to any of claims 1 to 4, **characterized in that** the embroidery on the side facing the top fabric (11) is made of a conductive yarn.

6. A device according to claim 5, **characterized in that** the conductive yarn comprises conductive metal wires, conductive metal fibre yarns, conductive synthetic materials, carbon materials or combinations and, respectively, mixtures thereof.

7. A device according to any of claims 1 to 6, **characterized in that** the textile top fabric (11) is configured so as to be hydrophilic, preferably being made of cotton, polyester or mixtures thereof.

8. A device according to any of claims 1 to 7, **characterized in that** the textile top fabric (11) is a knitted fabric, a woven fabric or a nonwoven.

9. A device according to any of claims 1 to 8, **characterized by** an additional measuring loop, the measuring loop preferably being applied as a reference section.

10. A device according to any of claims 1 to 9, **characterized by** electrical contact points (20) for the electrically conductive embroidery.

11. A device according to any of claims 1 to 10, **characterized in that** the bottom fabric (14) is a woven fabric, a knitted fabric or a nonwoven, the bottom fabric (14) preferably being a knitted polyester fabric.

12. A device according to any of claims 1 to 11 for moisture detection in textile structures, beds or diapers, **characterized by** an output unit for the presence of moisture.

13. A device according to any of claims 1 to 11 for determining the position of a subject in a bed, **characterized by** an output unit for changing the lying position of the subject.

14. A device according to claim 12 or claim 13, **characterized in that** the output unit is in radio communication with the sensor textile (1).

15. An arrangement comprising a device according to claim 12 or claim 14 for moisture detection and a device according to claim 13 or claim 14 for determining the position of a subject in a bed.

## Revendications

1. Dispositif comprenant un textile de détection (1), le textile de détection (1) étant constitué d'une structure multicouche, comprenant :
i) une couche supérieure constituée d'une matière textile supérieure (11),
ii) une couche textile intermédiaire (12) disposée en dessous de la couche supérieure, laquelle présente, sur le côté orienté en direction de la couche supérieure (11), des fils de détection (5) conducteurs, qui se présentent de préférence sous la forme d'une broderie,
iii) une couche faisant barrière (13) qui est disposée en dessous de la couche intermédiaire (12), et
iv) une couche inférieure constituée d'une matière textile inférieure (14) disposée sur le dessous, et une électronique d'analyse,
**caractérisé par** un dispositif de mesure qui permet de déterminer la résistance électrique à l'intérieur du textile de détection (1) et qui permet de déterminer l'impédance à l'intérieur du textile de détection (1), l'électronique d'analyse permettant de déterminer la partie réelle et la partie imaginaire de la résistance du textile de détection (1) à l'aide de la mesure d'impédance.

2. Dispositif selon la revendication 1, **caractérisé en ce que** le contre-fil qui est situé sur le côté orienté en direction de la couche faisant barrière (13) est constitué d'un fil textile.

3. Dispositif selon la revendication 1 ou la revendication 2, **caractérisé en ce que** la couche faisant barrière (13) est perméable à la vapeur d'eau et est sensiblement imperméable à l'eau liquide.

4. Dispositif selon l'une quelconque des revendications 1 à 3, **caractérisé en ce que** la broderie qui est située sur le côté orienté en direction de la matière supérieure (11) est sensiblement en forme de peigne.

5. Dispositif selon l'une quelconque des revendications 1 à 4, **caractérisé en ce que** la broderie située sur le côté orienté en direction de la matière supérieure (11) est constituée d'un fil conducteur.

6. Dispositif selon la revendication 5, **caractérisé en ce que** le fil conducteur comprend des fils métalliques conducteurs, des fils de fibres métalliques conducteurs, des matières synthétiques conductrices, des matériaux carbonés ou des combinaisons ou des mélanges de ceux-ci.

7. Dispositif selon l'une quelconque des revendications 1 à 6, **caractérisé en ce que** la matière textile supérieure (11) est hydrophile, de préférence en coton, en polyester ou en mélanges de ceux-ci.

8. Dispositif selon l'une quelconque des revendications 1 à 7, **caractérisé en ce que** la matière textile supérieure (11) est un textile tricoté, un textile tissé ou un textile non tissé.

9. Dispositif selon l'une quelconque des revendications 1 à 8, **caractérisé par** une boucle de mesure supplémentaire, la boucle de mesure étant de préférence appliquée en tant que segment de référence.

10. Dispositif selon l'une quelconque des revendications 1 à 9, **caractérisé par** des points de contact électrique (20) pour la broderie électriquement conductrice.

11. Dispositif selon l'une quelconque des revendications 1 à 10, **caractérisé en ce que** la matière inférieure (14) est un textile tissé, un textile tricoté ou un textile non tissé, la matière inférieure (14) étant de préférence un textile tricoté en polyester.

12. Dispositif selon l'une quelconque des revendications 1 à 11 pour permettre de détecter l'humidité à l'intérieur de structures textiles, de lits ou de couches, **caractérisé par** une unité de sortie pour la présence d'humidité.

13. Dispositif selon l'une quelconque des revendications 1 à 11 pour permettre de déterminer la position d'un sujet dans un lit, **caractérisé par** une unité de sortie pour modifier la position du sujet.

14. Dispositif selon la revendication 12 ou la revendication 13, **caractérisé en ce que** l'unité de sortie est en communication radio avec le textile de détection (1).

15. Agencement, comprenant un dispositif selon la revendication 12 ou la revendication 14 pour permettre de détecter l'humidité et un dispositif selon la revendication 13 ou la revendication 14 pour permettre de déterminer la position d'un sujet dans un lit.
